(19) Europäisches Patentamt / European Patent Office / Office européen des brevets

(11) **EP 4 442 774 A1**

(12) **EUROPEAN PATENT APPLICATION**
published in accordance with Art. 153(4) EPC

(43) Date of publication:
**09.10.2024 Bulletin 2024/41**

(21) Application number: **22898837.4**

(22) Date of filing: **12.10.2022**

(51) International Patent Classification (IPC):
**C09D 11/32** (2014.01)   **C09D 11/033** (2014.01)
**C09D 11/10** (2014.01)   **B41M 5/00** (2006.01)
**A23P 20/00** (2016.01)   **A61K 8/18** (2006.01)

(52) Cooperative Patent Classification (CPC):
**A23P 20/00; A61K 8/18; B41M 5/00; C09D 11/033;
C09D 11/10; C09D 11/32**

(86) International application number:
**PCT/KR2022/015375**

(87) International publication number:
**WO 2023/096154 (01.06.2023 Gazette 2023/22)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **25.11.2021 KR 20210164872**

(71) Applicant: **Activon Co., Ltd.**
**Cheongju-si, Chungcheongbuk-do 28104 (KR)**

(72) Inventors:
• **CHO, Se Hee**
 **Seoul 07691 (KR)**
• **KIM, Dae Gun**
 **Hanam-si, Gyeonggi-do 12942 (KR)**
• **CHO, Youn Ki**
 **Yongin-si, Gyeonggi-do 17005 (KR)**

(74) Representative: **Tiburzi, Andrea et al**
**Barzanò & Zanardo Roma S.p.A.**
**Via Piemonte 26**
**00187 Roma (IT)**

(54) **INK COMPOSITION FOR SURFACE PRINTING ON COSMETICS, AND METHOD FOR SURFACE PRINTING ON COSMETICS BY USING SAME**

(57)  The present application relates to an ink composition for printing the surface of a cosmetic and a method of printing the surface of a cosmetic by using the same, wherein the ink composition improves a caking phenomenon and enhances usability as an inkjet printing technology, and the method of printing the surface of a cosmetic improves printability, such as adhesion to the surface of the cosmetic, tinting strength or color development.

FIG. 2
BEFORE BRUSHING

AFTER BRUSHING

**Description**

Technical Field

The present disclosure relates to an ink composition for printing the surface of a cosmetic and a method of printing the surface of a cosmetic by using the same, and the present patent application claims priority to Korean Patent Application No. 10-2021-0164872, filed on November 25, 2021 before the Korean Intellectual Property Office, the entire contents of which are hereby incorporated by reference.

Background Art

Among makeup products, the design of products that users carry with them when going out is particularly important. Such products include solid powder cosmetics such as compacts, eye shadows, and the like, and cosmetics mainly based on oils and waxes, such as lipsticks, lip gloss, and skin covers. Therefore, for these products, there has been a constant demand for designs or decorations that may meet the needs of consumers.
As one such design or decoration, a method of adding characters or images to the surface of cosmetics has been proposed. However, a conventional method of representing characters or images by using embossed or intagliated punches or molds has technical limitations in that the characters or images to be represented are inevitably extremely simple, and represent only embossings and intaglios rather than colors. In addition, the conventional method of engraving the desired characters or images to a certain thickness by using a laser and then printing the characters or images by using a spray method is not easy to implement in the case of cosmetics that are sensitive to heat, and fundamentally has limitations in that various colors cannot be implemented.
In particular, face makeup and eye makeup products containing a large amount of powder necessarily use oil with a high refractive index and high viscosity to maximize gloss and enhance the adhesion of the powder to the skin. Accordingly, when water-soluble pigment materials are additionally applied to the surface of a composition containing oil with a high refractive index and viscosity, a caking phenomenon occurs in which the surface becomes hard and accordingly it is difficult to take off the powder, causing a problem of reduced pay-off. In particular, when performing a process of printing designs containing various water-soluble pigments on the surface of makeup products, there is a risk that the powder and oil will clump together, further worsening the caking phenomenon mentioned above.
Against this technical background, various studies are being conducted on natural/synthetic ink compositions that may be utilized on the surface of cosmetics, for example, cosmetics of a solid powder formulation, and surface printing technology for cosmetics (Korean Patent No. 10-2035916), but are still insufficient.

Disclosure

Technical Problem

To solve the problems described above, the present inventors developed an ink composition for printing the surface of a cosmetic and a method of printing the surface of a cosmetic by using the same, and according to the ink composition or method, the present disclosure was completed by confirming that vivid full-color printing may be implemented, as well as the caking phenomenon caused by agglomeration between water-soluble pigments and oil components in the cosmetic may be improved.
One object of the present disclosure is to provide an ink composition for printing the surface of a cosmetic, which enhances usability as an inkjet printing technology.
Another object of the present disclosure is to provide a cosmetic composition of which the surface is printed with the ink composition.
Another object of the present disclosure is to provide a method of printing the surface of a cosmetic by using the ink composition.
Another object of the present disclosure is to provide a cosmetic composition of which the surface is printed by the method of printing the surface of a cosmetic.
Another object of the present disclosure is to provide a method of preparing the ink composition.
Another object of the present disclosure is to provide use of the ink composition for printing the surface of a cosmetic.
Another object of the present disclosure is to provide use of the ink composition for preparing an ink for printing the surface of a cosmetic.
However, the technical problem to be solved by the present disclosure is not limited to the problems mentioned above, and another problems not mentioned will may be clearly understood by those skilled in the art from the description below.

Technical Solution

Unless otherwise defined, all technical and scientific terms used herein have the same meaning as commonly understood by a person skilled in the art to which the present disclosure pertains. In general, the nomenclature used herein is well known and commonly used in the art.

According to one embodiment, provided is an ink composition for printing the surface of a cosmetic, the ink composition including: a water-soluble pigment; a water-miscible organic solvent; and a surfactant.

As used herein, the term "ink composition (composition of ink)" refers to a printing composition including naturally derived pigments or artificially combined and prepared pigments. For the purpose of the present disclosure, the ink composition, unlike ink compositions in other fields, is used for design or decorative purposes, and has to be applied to the surface of a desired material, for example, the surface of a cosmetic of a solid powder formulation, to develop unique colors derived from pigments, but the ink composition has to be removed from the surface of the cosmetic through contacts such as brushing and the like, so that the functionality of a cosmetic base located under the ink composition may be effectively achieved.

In one embodiment, the ink composition may be printed on the surface of a cosmetic, and preferably, may be applied to the surface of a cosmetic by using an inkjet printing method. Here, the surface of a cosmetic refers to the upper side of a base component constituting the cosmetic, or to the side exposed to the outside relative to the user. Additionally, the inkjet printing method may use a piezo method or a thermal method. For this purpose, the ink composition requires jetting stability during printing, strong color development or tinting strength, storage stability, biostability, and the like.

In one embodiment, the ink composition may be printed on the surface of a cosmetic and then removed from the surface of the cosmetic through 4 to 10 contacts. Therefore, the ink composition has to possess physical properties so that the ink composition may be easily removed from the surface of a cosmetic of a solid powder formulation or an oil-based cosmetic while having excellent color development and the like, as described above. However, when water-soluble pigment materials are applied to the surface of a cosmetic composition containing oil with high refractive index and viscosity, a caking phenomenon may be caused in which the powder components present on the surface of the cosmetic composition become hard and it is difficult to take the powders. This caking phenomenon acts as a major obstacle in implementing the present technology, and specifically, it not only causes the ink to spread, but also may have a negative effect on the functionality of the cosmetic base located under the ink composition.

In one embodiment, the surfactant is for improving the caking phenomenon caused by agglomeration between water-soluble pigments and oil components in a cosmetic and improving stability under sealed/room temperature dry conditions and flowability in the nozzle, wherein the surfactant may be a glyceryl ester-based surfactant (or a glyceryl fatty acid ester-based surfactant), a silicone-based surfactant, or a combination thereof. Specifically, the glyceryl ester-based surfactant (or the glyceryl fatty acid ester-based surfactant) may be any one selected from the group consisting of polyglyceryl-10 pentahydroxystearate, polyglyceryl-6 polyricinoleate, polyglycerin-10 hexaerucate, polyglyceryl-6 ricinolate, polyglyceryl-4 isostearate, polyglyceryl-3 diisostearate, polyglyceryl-4 diisostearate/polyhydroxystearate/sebacate, polyglyceryl-2 diisostearate, glyceryl isostearate, and combinations thereof, but is not limited thereto. In addition, the silicone-based surfactant may be any one selected from the group consisting of a cetyl PEG/PPG-10/1 dimethicone, a PEG-10 dimethicone, a PEG-9 polydimethylsiloxyethyl dimethicone, a polyglyceryl-3 polydimethylsiloxyethyl dimethicone, and combinations thereof, but is not limited thereto. The surfactant may be a glyceryl ester-based nonionic surfactant (or a glyceryl fatty acid ester-based nonionic surfactant), a silicone-based nonionic surfactant, or a combination thereof.

According to one embodiment, when the ink composition includes a glyceryl ester-based surfactant (or a glyceryl fatty acid ester-based surfactant), a silicone-based surfactant, or a combination thereof as a surfactant, the ink composition may have significantly improved adhesion to the surface of a cosmetic, tinting strength, color development, or printability, and at the same time, in actually using the cosmetic with the ink composition printed on the surface thereof, the ink composition may be easily separated or removed from the surface of the cosmetic, which may not cause the caking phenomenon. On the other hand, according to one embodiment, when the ink composition includes a surfactant other than the glyceryl ester-based surfactant (or the glyceryl fatty acid ester-based surfactant) and the silicone-based surfactant (such as Surfynol465, Polysorbate 20, etc.), in actually using the cosmetic with the ink composition printed on the surface thereof, the caking phenomenon may be caused, thereby deteriorating the feeling of use and quality of the cosmetic. Therefore, when printing the surface of a cosmetic by using the ink composition including the glyceryl ester-based surfactant (or the glyceryl fatty acid ester-based surfactant), the silicone-based surfactant, or a combination thereof, the problems of feeling of use and quality deterioration by the caking phenomenon caused in existing surface-printed cosmetics may be solved.

In one embodiment, the surfactant may have a hydrophilic lipophilic balance (HLB) value of 3 to 8. Specifically, the surfactant may have a HLB value of 3 to 7, 3 to 6, or 3 to 5. According to one embodiment, when the HLB value of a surfactant included in the ink composition is outside the above numerical range, in actually using the cosmetic with the ink composition printed on the surface thereof, the caking phenomenon may be caused, thereby deteriorating the feeling of use and quality of the cosmetic, and when the HLB value of a surfactant included in the ink composition is within the above numerical range, in actually using the cosmetic with the ink composition printed on the surface thereof, the ink composition may be easily separated or removed from the surface of the cosmetic, which may not cause the caking phenomenon. Therefore, when printing the surface of a cosmetic by using the ink composition including a surfactant with a HLB value of 3 to 8, the problems of feeling of use and quality deterioration by the caking phenomenon caused in existing surface-printed cosmetics may be solved.

The HLB value is a value used as a measure of the degree of hydrophilicity and lipophilicity of a surfactant. Definitions and calculation methods of HLB values are described in, for example, W. C. Griffin, J. Soc. Cosmetic Chem., volume 5,249 (1954), and K. H. Schrader, Grundlagen und Rezepturen der Kosmetika [Fundamentals and formulations of cosmetics], Huethig Verlag, 2nd edition, 1989. For example, the Griffin formula for calculating the HLB values for nonionic surfactants developed in 1954 is as follows:

$$HLB = 20 * M_h/M.$$

Here, $M_h$ is the molecular mass of the hydrophilic part of the molecule, and M is the mass of the entire molecule. When calculated by using this formula, a value between 0 and 20 is obtained. When the HLB value is completely 0, it means that the surfactant is a completely lipophilic/hydrophobic molecule, and when the HLB value is 20, it means that the surfactant is a completely hydrophilic/oleophobic molecule.

In one embodiment, the surfactant may be included in an amount of 0.05 to 0.5 wt% based on a total weight of the ink composition. The content of the surfactant may be, for example, 0.05 to 0.45 wt%, 0.05 to 0.4 wt%, 0.05 to 0.35 wt%, 0.05 to 0.3 wt%, 0.05 to 0.25 wt%, 0.05 to 0.2 wt%, 0.05 to 0.15 wt%, 0.1 to 0.5 wt%, 0.1 to 0.45 wt%, 0.1 to 0.4 wt%, 0.1 to 0.35 wt%, 0.1 to 0.3 wt%, 0.1 to 0.25 wt%, 0.1 to 0.2 wt%, 0.1 to 0.15 wt%, 0.2 to 0.5 wt%, 0.2 to 0.45 wt%, 0.2 to 0.4 wt%, 0.2 to 0.35 wt%, 0.2 to 0.3 wt%, or 0.2 to 0.25 wt%. When the content of the surfactant is too small or too much outside the above range, there is a risk that the caking phenomenon may be caused, and thus the quality of a cosmetic may deteriorate, or effective color development/tinting effects may not be achieved.

As used herein, the term "water-miscible organic solvent" refers to an organic solvent that may be uniformly mixed with water in an appropriate concentration range. In one embodiment, the water-miscible organic solvent may be any one selected from the group consisting of an alkylene glycol having 2 to 5 carbon atoms, a glycerin, a monoalcohol having 1 to 5 carbon atoms, an ether of an alkylene glycol having 2 to 5 carbon atoms, an ester of an alkylene glycol having 2 to 5 carbon atoms, a monooxyalkylenediol having 2 to 5 carbon atoms, a polyoxyalkylenediol having 5 to 10 carbon atoms, a thiodiglycol, a monoalkanolamine, a dialkanolamine, a trialkanolamine, and combinations thereof. The monoalcohol having 1 to 5 carbon atoms may be, for example, a methanol, an ethanol, an isopropanol, a butanol, a pentanol, or the like, and the alkylene glycol having 2 to 5 carbon atoms may be, for example, an ethylene glycol, a propylene glycol, a butanediol, or the like. In addition, the monooxyalkylenediol having 2 to 5 carbon atoms may be, for example, diethyleneglycols, and the polyoxyalkylenediol having 5 to 10 carbon atoms may be, for example, a polyethyleneglycol.

In one embodiment, the water-soluble pigment may include both a natural pigment or an artificially prepared synthetic pigment, for example, Sunset Yellow FCF, New Coccine, Brilliant Blue FCF, and combinations thereof, but may be applied in a nonlimiting way as long as it can exert the function of developing color and/or tinting. The natural pigment may be obtained by extracting and refining one or more natural materials, for example, selected from the group consisting of mulberry fruit, madder, red beet, strawberry, cactus fruit, gardenia red, wine, grape skin, red cabbage, pomegranate, garden balsam, borage, prickly pear, safflower red, Biancaea sappan, Lithospermum root, Lithospermum erythrorhizon, blueberry, gallnut, annatto, Mallotus japonicus, acacia, Santalum album, Betula platyphylla, phellodendron amurense Ruprecht, Coptis chinensis, Sophorae Flos, turmeric, gardenia yellow, saffron, safflower yellow, marigold, red yeast rice yellow, Rehmannia glutinosa, bitter gourd, onion skin, persimmon, clove, chestnut, rhubarb, gambir, Rhamnus davurica, mulberry leaves, spinach, spirulina, chlorella, Capsosiphon fulvescens, mugwort, gardenia green, and indigo fruit. Additionally, the synthetic pigment may be a combination of the components shown in Table 1 below, but is not limited thereto.

[Table 1]

| Serial number | Pigment |
| --- | --- |
| 1 | Green No. 204 (Pyranine Conc)* CI 59040 Trisodium salt of 8-hydroxy-1, 3, 6-pyrenetrisulfonic acid |

(continued)

| Serial number | Pigment |
|---|---|
| 2 | Green No. 401 (Naphthol Green B)* CI 10020 Iron salt of 5-isonitroso-6-oxo-5,6-dihydro-2-naphthalenesulfonic acid |
| 3 | Orange No. 206 (Diiodofluorescein)* CI 45425:1 4', 5'-diiodo-3', 6'-dihydroxyspiro[isobenzofuran-1 (3H), 9'-[9H]xanthene]-3-one |
| 4 | Orange No. 207 (Erythrosine Yellowish NA)* CI 45425 Disodium salt of 9-(2-carboxyphenyl)-6-hydroxy-4,5- diiodo-3H-xanthen-3-one |
| 5 | Purple No. 401 (Alizurol Purple)* CI 60730 Monosodium salt of 1-hydroxy-4-(2-sulfo-p-toluino)-anthraquinone |
| 6 | Red No. 205 (Lithol Red)* CI 15630 Monosodium salt of 2-(2-hydroxy-1-naphthylazo)-1-naphthalenesulfonic acid |
| 7 | Red No. 206 (Lithol Red CA)* CI 15630:2 Calcium salt of 2-(2-hydroxy-1-naphthylazo)-1-naphthalenesulfonic acid |
| 8 | Red No. 207 (Lithol Red BA) CI 15630:1 Barium salt of 2-(2-hydroxy-1-naphthylazo)-1-naphthalenesulfonic acid |
| 9 | Red No. 208 (Lithol Red SR) CI 15630:3 Strontium salt of 2-(2-hydroxy-1-naphthylazo)-1-naphthalenesulfonic acid |
| 10 | Red No. 219 (Brilliant Lake Red R)* CI 15800 Calcium salt of 3-hydroxy-4-phenylazo-2-naphthoic acid |
| 11 | Red No. 225 (Sudan III)* CI 26100 1-[4-(phenylazo)phenylazo]-2-naphthol |
| 12 | Red No. 405 (Permanent Red F5R) CI 15865:2 Calcium salt of 4-(5-chloro-2-sulfo-p-tolylazo)-3-hydroxy-2-naphthoic acid |
| 13 | Red No. 504 (Ponceau SX)* CI 14700 Disodium salt of 2-(5-sulfo-2,4-xylylazo)-1-naphthol-4-sulfonic acid |
| 14 | Blue No. 404 (Phthalocyanine Blue)* CI 74160 Copper complex salt of phthalocyanine |
| 15 | Yellow No. 202(2) (Uranine K)* CI 45350 Dipotassium salt of 9-ortho-carboxyphenyl-6-hydroxy-3-isoxanthone |
| 16 | Yellow No. 204 (Quinoline Yellow SS)* CI 47000 2-(2-quinolyl)-1, 3-indandione |
| 17 | Yellow No. 401 (Hanza Yellow)* CI 11680 N-phenyl-2-(nitro-p-tolylazo)-3-oxobutanamide |
| 18 | Yellow No. 403(1) (Naphthol Yellow S) CI 10316 Disodium salt of 2,4-dinitro-1-naphthol-7-sulfonic acid |
| 19 | Orange No. 205 (Orange II) CI 15510 Monosodium salt of 1-(4-sulfophenylazo)-2-naphthol |
| 20 | Yellow No. 203 (Quinoline Yellow WS) CI 47005 Sodium salts of 2-(1, 3-dioxoindan-2-yl)quinoline monosulfonic acid and disulfonic acid |
| 21 | Green No. 3 (Fast Green FCF) CI 42053 Disodium salt of 2-[$\alpha$-[4-(N-ethyl-3-sulfobenzyliminio)-2,5-cyclohexadienyldene]-4-(N-ethyl-3-sulfobenzylamino)benzyl]-5-hydroxybenzenesulfonate |
| 22 | Green No. 201 (Alizarine Cyanine Green F)* CI 61570 Disodium salt of 1, 4-bis-(2-sulfo-p-toluidino)-anthraquinone |
| 23 | Green No. 202 (Quinizarine Green SS)* CI 61565 1, 4-bis(p-toluidino)anthraquinone |
| 24 | Orange No. 201 (Dibromofluorescein) CI 45370:1 4', 5'-dibromo-3', 6'-dihydroxyspiro[isobenzofuran-1(3H),9-[9H]xanthen-3-one |
| 25 | Purple No. 201 (Alizurine Purple SS)* CI 60725 1-hydroxy-4-(p-toluidino)anthraquinone |
| 26 | Red No. 2 (Amaranth) C! 16185 |
|  | Trisodium salt of 3-hydroxy-4-(4-sulfonaphthylazo)-2, 7-naphthalenedisulfonic acid |

(continued)

| Serial number | Pigment |
|---|---|
| 27 | Red No. 40 (Allura Red AC) CI 16035 Disodium salt of 6-hydroxy-5-[(2-methoxy-5-methyl-4-sulfophenyl)azo]-2-naphthalenesulfonic acid |
| 28 | Red No. 1 02 (New Coccine) CI 16255 1.5 hydrate of trisodium salt of 1-(4-sulfo-1-naphthylazo)-2-naphthol-6, 8-disulfonic acid |
| 29 | Red No. 1 03(1) (Eosine YS) CI 45380 Disodium salt of 9-(2-carboxyphenyl)-6-hydroxy-2, 4, 5, 7-tetrabromo-3H-xanthen-3-one |
| 30 | Red No. 1 04(1) (Phloxine B) CI 45410 Disodium salt of 9-(3, 4, 5, 6-tetrachloro-2-carboxyphenyl)-6-hydroxy-2, 4, 5, 7-tetrabromo-3H-xanthen-3-one |
| 31 | Red No. 1 04(2) (Phloxine BK) CI 45410 Dipotassium salt of 9-(3, 4, 5, 6-tetrachloro-2-carboxyphenyl)-6-hydroxy-2, 4, 5, 7-tetrabromo-3H-xanthen-3-one |
| 32 | Red No. 201 (Lithol Rubine B) CI 15850 Disodium salt of 4-(2-sulfo-p-tolylazo)-3-hydroxy-2-naphthoic acid |
| 33 | Red No. 202 (Lithol Rubine BCA) CI 15850:1 Calcium salt of 4-(2-sulfo-p-tolylazo)-3-hydroxy-2-naphthoic acid |
| 34 | Red No. 218 (Tetrachlorotetrabromofluorescein) CI 45410:1 2', 4', 5', 7'-tetrabromo-4, 5, 6, 7-tetrachloro-3', 6'-dihydroxypyro[isobenzofuran-1(3H),9'-[9H]xanthene]-3-one |
| 35 | Red No. 220 (Deep Maroon)* CI 15880:1 Calcium salt of 4-(1-sulfo-2-naphthylazo)-3-hydroxy-2-naphthoic acid |
| 36 | Red No. 223 (Tetrabromofluorescein) CI 45380:2 2', 4', 5', 7'-tetrabromo-3', 6'-dihydroxyspiro[isobenzofuran-1(3H),9'-[9H]xanthene]-3-one |
| 37 | Red No. 226 (Helindone Pink CN)* CI 73360 6, 6'-dichloro-4, 4'-dimethyl-thioindigo |
| 38 | Red No. 227 (Fast Acid Magenta)* CI 17200 Disodium salt of 8-amino-2-phenylazo-1-naphthol-3, 6-disulfonic acid |
| 39 | Red No. 228 (Permaton Red) CI 12085 1-(2-chloro-4-nitrophenylazo)-2-naphthol |
| 40 | Red No. 230(2) (Eosine YSK) CI 45380 Dipotassium salt of 9-(2-carboxyphenyl)-6-hydroxy-2, 4, 5, 7-tetrabromo-3H-xanthen-3-one |
| 41 | Blue No. 1 (Brilliant Blue FCF) CI 42090 Disodium salt of 2-[α-[4-(N-ethyl-3-sulfobenzyliminio)-2,5-cyclohexadienylidene]-4-(N-ethyl-3-sulfobenzylamino)benzyl]benzenesulfonate |
| 42 | Blue No. 2 (Indigo Carmine) CI 73015 |
| | Disodium salt of 5, 5'-indigotine disulfonic acid |
| 43 | Blue No. 201 (Indigo)* CI 73000 Indigotine |
| 44 | Blue No. 204 (Carbanthrene Blue)* CI 69825 3, 3'-dichloroindanthrene |
| 45 | Blue No. 205 (Alphazurine FG)* CI 42090 Diammonium salt of 2-[α-[4-(N-ethyl-3-sulfobenzyliminio)-2,5-cyclohexanedienylidene]-4-(N-ethyl-3-sulfobenzylamino)benzyl]benzenesulfonate |
| 46 | Yellow No. 4 (Tartrazine) C! 19140 Trisodium salt of 5-hydroxy-1-(4-sulfophenyl)-4-(4-sulfophenylazo)-1H-pyrazole-3-carboxylic acid |
| 47 | Yellow No. 5 (Sunset Yellow FCF) C! 15985 Disodium salt of 6-hydroxy-5-(4-sulfophenylazo)-2-naphthalenesulfonic acid |
| 48 | Yellow No. 201 (Fluorescein)* CI 45350:1 3', 6'-dihydroxyspiro[isobenzofuran-1(3H), 9'-[9H]xanthene]-3-one |
| 49 | Yellow No. 202(1) (Uranine)* CI 45350 Disodium salt of 9-(2-carboxyphenyl)-6-hydroxy-3H-xanthen-3-one |

(continued)

| Serial number | Pigment |
|---|---|
| 50 | Orange No. 204 (Benzidine Orange G)* CI 21110 4, 4'-[(3, 3'-dichloro-1, 1'-biphenyl)-4, 4'-diylbis (azo)]bis[3-methyl-1-phenyl-5-pyrazolone] |
| 51 | Red No. 1 06 (Acid Red)* CI 45100 Monosodium salt of 2-[[N, N-diethyl-6-(diethylamino)-3H-xanthene-3-iminio]-9-yl]-5-sulfobenzenesulfonate |
| 52 | Red No. 221 (Toluidine Red)* C! 12120 1-(2-nitro-p-tolylazo)-2-naphthol |
| 53 | Red No. 401 (Violamine R) CI 45190 Disodium salt of 9-(2-carboxyphenyl)-6-(4-sulfo-ortho-toluidino)-N-(ortho-tolyl)-3H-xanthene-3-imine |
| 54 | Red No. 506 (Fast Red S)* CI 15620 Monosodium salt of 4-(2-hydroxy-1-naphthylazo)-1-naphthalenesulfonic acid |
| 55 | Yellow No. 407 (Fast Light Yellow 3G)* C! 18820 Monosodium salt of 3-methyl-4-phenylazo-1-(4-sulfophenyl)-5-pyrazolone |
| 56 | Black No. 401 (Naphthol Blue Black)* CI 20470 Disodium salt of 8-amino-7-(4-nitrophenylazo)-2-(phenylazo)-1-naphthol-3, 6-disulfonic acid |
| 57 | Orange No. 401 (Orange no. 401)* CI 11725 |
| 58 | Annatto CI 75120 |
| 59 | Lycopene CI 75125 |
| 60 | Beta-Carotene CI 75130 |
| 61 | Guanine (2-Amino-1,7-dihydro-6H-purin-6-one) CI 75170 |
| 62 | Curcumin CI 75300 |
| 63 | Carmines CI 75470 |
| 64 | Chlorophylls CI 75810 |
| 65 | Aluminum CI 77000 |
| 66 | Bentonite CI 77004 |
| 67 | Ultramarines CI 77007 |
| 68 | Barium sulfate CI 77120 |
| 69 | Bismuth oxychloride CI 77163 |
| 70 | Calcium carbonate CI 77220 |
| 71 | Calcium sulfate CI 77231 |
| 72 | Carbon black CI 77266 |
| 73 | Bone black (Bone charcoal) CI 77267 |
| 74 | Vegetable Carbon (Coke Black) CI 77268:1 |
| 75 | Chromium Oxide Greens (Chromium(III) oxide) CI 77288 |
| 76 | Chromium Hydroxide Green (Chromium(III) hydroxide) CI 77289 |
| 77 | Cobalt aluminum oxide CI 77346 |
| 78 | Copper CI 77400 |
| 79 | Gold CI 77480 |
| 80 | Ferrous oxide (Iron oxide) CI 77489 |
| 81 | Red iron oxide (Iron oxide red, ferric oxide) CI 77491 |
| 82 | Yellow iron oxide (Iron oxide yellow, hydrated ferric oxide) CI 77492 |

(continued)

| Serial number | Pigment |
|---|---|
| 83 | Black iron oxide (Iron oxide black, ferrous-ferric oxide) CI 77499 |
| 84 | Ferric ammonium ferrocyanide CI 77510 |
| 85 | Ferric ferrocyanide CI 77510 |
| 86 | Magnesium carbonate CI 77713 |
| 87 | Manganese Violet (Ammonium manganese(3+) diphosphate) CI 77742 |
| 88 | Silver CI 77820 |
| 89 | Titanium dioxide CI 77891 |
| 90 | Zinc oxide CI 77947 |
| 91 | Riboflavin (Lactoflavin) |
| 92 | Caramel |
| 93 | Paprika extract, capsanthin/capsorubin |
| 94 | Beetroot Red |
| 95 | Anthocyanins (Cyanidin, peonidin, malvidin, delphinidin, petunidin, peragonidin) |
| 96 | Aluminum stearate/zinc stearate/magnesium stearate/ calcium stearate |
| 97 | Disodium EDTA-copper |
| 98 | Dihydroxyacetone |
| 99 | Guaiazulene |
| 100 | Pyrophyllite |
| 101 | Mica CI 77019 |
| 102 | Bronze |
| 103 | Basic Brown 16 CI 12250 |
| 104 | Basic Blue 99 CI 56059 |
| 105 | Basic Red 76 CI 12245 |
| 106 | Basic Brown 17 CI12251 |
| 107 | Basic Yellow 87 |
| 108 | Basic Yellow 57 CI 12719 |
| 109 | Basic Red 51 |
| 110 | Basic Orange 31 |
| 111 | HC Blue No. 15 |
| 112 | HC Blue No. 16 |
| 113 | Disperse Violet 1 CI 61100 1,4-diaminoanthraquinone |
| 114 | HC Red No. 1 4-amino-2-nitrodiphenylamine |
| 115 | 2-amino-6-chloro-4-nitrophenol |
| 116 | 4-hydroxypropyl amino-3-nitrophenol |
| 117 | Basic Violet 2 CI 42520 |
| 118 | Disperse Black 9 |
| 119 | HC Yellow No. 7 |

(continued)

| Serial number | Pigment |
|---|---|
| 120 | Acid Red 52 CI 45100 |
| 121 | Acid Red 92 |
| 122 | HC Blue 17 |
| 123 | HC Orange No. 1 |
| 124 | Disperse Blue 377 |
| 125 | HC Blue No. 12 |
| 126 | HC Yellow No. 17 |
| 127 | Pigment Red 5* CI 12490 N-(5-chloro-2,4-dimethoxyphenyl)-4-[[5-[(diethylamino)sulfonyl]-2-methoxyphenyl]azo]-3-hydroxynaphthalene-2-carboxamide |

In one embodiment, the ink composition may include 2 to 7 wt% of a water-soluble pigment, 10 to 25 wt% of a water-miscible organic solvent, and 0.05 to 0.5 wt% of a surfactant, based on a total weight of the ink composition. Here, the content of the water-soluble pigment may be 2 to 6.5 wt%, 2 to 6.5 wt%, 2 to 5.5 wt%, 2 to 5.0 wt%, 2 to 4.5 wt%, 2 to 4.0 wt%, 2 to 3.5 wt%, 2 to 3.0 wt%, 2 to 2.5 wt%, 3 to 7 wt%, 3 to 6.5 wt%, 3 to 6.5 wt%, 3 to 5.5 wt%, 3 to 5.0 wt%, 3 to 4.5 wt%, 3 to 4.0 wt%, or 3 to 3.5 wt%, the content of the water-miscible organic solvent may be 10 to 23 wt%, 10 to 21 wt%, 10 to 19 wt%, 10 to 17 wt%, 10 to 15 wt%, 10 to 13 wt%, 10 to 11 wt%, 12 to 25 wt%, 12 to 23 wt%, 12 to 21 wt%, 12 to 19 wt%, 12 to 17 wt%, 12 to 15 wt%, or 12 to 13 wt%, and the content of the surfactant is as described above. When the contents are too small or too much outside the above range, the color development, tinting strength, or printed color density may be significantly reduced, and the original colors, formulations, or feeling of use of a cosmetic may be affected by the ink composition printed on the surface of the cosmetic.

In addition, the ink composition may further include distilled water, vegetable extracts, or various stabilizers (antifoaming agents, emulsifiers, humectants, thickeners, stabilizers, anti-settling agents, acidity regulators, preservatives, etc.) in addition to the components mentioned above, and the type and content thereof may be suitably changed depending on the purpose of use.

In one embodiment, the cosmetic to which the ink composition is applied may be, for example, a solid cosmetic formulation, a solid powder cosmetic formulation, an oil-based cosmetic formulation, a cream cosmetic formulation, or a gel cosmetic formulation, but is not limited thereto. Specifically, the cosmetic may have a formulation such as compact, powder pact, foundation, cushion compact, powder blusher, cream blusher, eye shadow, lipstick, sun stick, lip balm, soap, wax, or the like, but is not limited thereto. The cosmetic to which the ink composition is applied may be, for example, a basic cosmetic formulation. Specifically, the cosmetic may have a formulation such as cream, essence, lotion, serum, toner, mask pack, sunscreen, moisture gel, or the like, but is not limited thereto. The ink composition may be used to print not only the surface of cosmetics, but also the surface of food, medicine, and the like.

According to one example, when the surface of a cosmetic was printed by using the ink composition having the above-mentioned composition and content, it was confirmed that vivid full-color printing was possible, and in particular, not only the caking phenomenon, which was pointed out as an existing problem, was reduced, but also the stability under sealed/room temperature dry conditions and the clogging phenomenon in the nozzle caused by the ink composition were significantly improved. Therefore, the ink composition of the present disclosure has enhanced usability as an inkjet printing technology and may be used for printing the surface of a cosmetic.

Another aspect provides a method of printing the surface of a cosmetic, comprising applying the ink composition to the surface of the cosmetic by using an inkjet printing method.

The method may further include treating the surface of the cosmetic with plasma before the applying.

As used herein, the term "plasma" refers to an ionized gas that satisfies the Debye sheath in the fields of physics or chemistry. The plasma has high electrical conductivity due to free charges and may have very high reactivity to electromagnetic fields. The plasma may be in a gaseous state in which ions, electrons, atoms, molecules, and the like are mixed. The plasma may emit light. The surface of a material may be modified by the plasma. Specifically, when the surface of a cosmetic is treated with the plasma, the oil-soluble cosmetic surface may be modified to be water-soluble.

In one embodiment, when printing the surface of a cosmetic by using the ink composition after treating the surface of the cosmetic with the plasma, the separation phenomenon between the ink composition and the surface of the cosmetic, tinting or color development deterioration, the agglomeration phenomenon, the phenomenon in which water droplets form, and the like may be significantly reduced. That is, by treating the surface of the cosmetic with the plasma, when printing the surface of the cosmetic by using the ink composition, the adhesion to the surface of the cosmetic, tinting strength, color development, or printability of the ink composition may be significantly improved.

In one embodiment, the plasma may be atmospheric pressure plasma, and preferably, the treating the surface of the cosmetic with plasma may be performed by a direct plasma treatment method. Here, the direct plasma treatment method is a method of directly irradiating plasma to the sample, and may be distinguished from a remote plasma treatment method of generating plasma in a reactor and then strongly blowing radicals onto the sample.

In one embodiment, when the surface of the cosmetic is treated with plasma by the direct plasma treatment method, the water-soluble modification of the oil-soluble cosmetic surface may be significantly increased compared to that in case of using the remote plasma treatment method, and accordingly, the adhesion to the surface of the cosmetic, tinting strength, color development, or printability of the ink composition may be significantly improved.

In one embodiment, the ink composition used in the method may include: a water-soluble pigment; a water-miscible organic solvent; and a surfactant. The composition, type, characteristics, and content of the above components are as described above. In one embodiment, the ink composition may be applied to the surface of a cosmetic and then removed from the surface of the cosmetic by 4 to 10 contacts.

In one embodiment, the cosmetic may be, for example, a solid cosmetic formulation, a solid powder cosmetic formulation, an oil-based cosmetic formulation, a cream cosmetic formulation, or a gel cosmetic formulation, but is not limited thereto. Specifically, the cosmetic may have a formulation such as compact, powder pact, foundation, cushion compact, powder blusher, cream blusher, eye shadow, lipstick, sun stick, lip balm, soap, wax, or the like, but is not limited thereto. The cosmetic may be, for example, a basic cosmetic formulation. Specifically, the cosmetic may have a formulation such as cream, essence, lotion, serum, toner, mask pack, sunscreen, moisture gel, or the like, but is not limited thereto. The method may be used to print not only the surface of cosmetics, but also the surface of food, medicine, and the like.

A method of printing the surface of the cosmetic may be performed by using an inkjet printer. The inkjet printer may use a piezo method or a thermal method.

Among the terms or elements mentioned in the above method, those mentioned in the description of the composition may be understood as the same as previously mentioned in the description of the composition.

Another aspect provides a cosmetic composition of which the surface is printed with the ink composition.

Another aspect provides a cosmetic composition of which the surface is printed by the method of printing the surface of the cosmetic.

In one embodiment, the cosmetic may be, for example, a solid cosmetic formulation, a solid powder cosmetic formulation, an oil-based cosmetic formulation, a cream cosmetic formulation, or a gel cosmetic formulation, but is not limited thereto. Specifically, the cosmetic may have a formulation such as compact, powder pact, foundation, cushion compact, powder blusher, cream blusher, eye shadow, lipstick, sun stick, lip balm, soap, wax, or the like, but is not limited thereto. The cosmetic may be, for example, a basic cosmetic formulation. Specifically, the cosmetic may have a formulation such as cream, essence, lotion, serum, toner, mask pack, sunscreen, moisture gel, or the like, but is not limited thereto.

Among the terms or elements mentioned in the cosmetic composition, those mentioned in the descriptions of the ink composition and method may be understood as the same as previously mentioned in the descriptions of the ink composition and method.

Another aspect provides a method of preparing an ink composition for printing the surface of a cosmetic, the method including: mixing a water-soluble pigment; a water-miscible organic solvent; and a surfactant, wherein the surfactant is a glyceryl ester-based surfactant (or a glyceryl fatty acid ester-based surfactant), a silicone-based surfactant, or a combination thereof. The composition, type, characteristics, and content of the above components are as described above.

Among the terms or elements mentioned in the method of preparing the ink composition, those mentioned in the description of the ink composition may be understood as the same as previously mentioned in the description of the ink composition.

Another aspect provides use of an ink composition for printing the surface of a cosmetic, the ink composition including: a water-soluble pigment; a water-miscible organic solvent; and a surfactant, wherein the surfactant is a glyceryl ester-based surfactant (or a glyceryl fatty acid ester-based surfactant), a silicone-based surfactant, or a combination thereof.

Another aspect provides use of an ink composition for preparing an ink for printing the surface of a cosmetic, the composition including: a water-soluble pigment; a water-miscible organic solvent; and a surfactant, wherein the surfactant is a glyceryl ester-based surfactant (or a glyceryl fatty acid ester-based surfactant), a silicone-based surfactant, or a combination thereof.

The composition, type, characteristics, and content of the above components are as described above.

Among the terms or elements mentioned in the above use, those mentioned in the descriptions of the ink composition and method may be understood as the same as previously mentioned in the descriptions of the ink composition and method.

## Advantageous Effects

According to the ink composition according to one aspect, not only vivid full-color printing may be implemented, but also the caking phenomenon caused by agglomeration between water-soluble pigments and oil components in a cosmetic may be improved. That is, the ink composition may improve the caking phenomenon when removed from the surface of the cosmetic after being printed on the surface of the cosmetic.

In addition, the ink composition according to one aspect may improve stability under sealed/room temperature dry conditions and flowability within the nozzle, and it was thus confirmed to be useful for printing the surface of a cosmetic composition through an inkjet printing method.

In addition, according to the method of printing the surface of a cosmetic according to one aspect, by treating the surface of the cosmetic with plasma before printing the surface, the printability of the ink composition, such as adhesion to the surface of the cosmetic, tinting, or color development, may be significantly improved.

## Description of Drawings

FIG. 1 is results of printing the surface of a cosmetic by using an ink composition according to one example and then visually confirming the same.

FIG. 2 is results of printing the surface of a cosmetic by using an ink composition according to one example, brushing five times, and then visually confirming the presence of patterns and a caking phenomenon due to the ink composition.

FIG. 3 is results of printing the surface of a cosmetic by using an ink composition of a comparative example, brushing five times, and then visually confirming the presence of patterns and a caking phenomenon by the ink composition.

FIG. 4 is results of confirming the water-soluble modification of the oil-soluble cosmetic surface by atmospheric pressure plasma treatment.

FIG. 5 is results of visually confirming the surface of a cosmetic after printing the surface of the cosmetic by using the ink composition before (top) and after (bottom) plasma treatment.

FIG. 6 is a schematic diagram schematically showing the difference between direct plasma and remote plasma.

## Best Mode

## Mode for Invention

Hereinafter, the present disclosure will be described in more detail through examples. However, these examples are for illustrative purposes only and the scope of the present disclosure is not limited to these examples.

## Example 1. - Example 8. Preparation of ink composition for printing surface of cosmetic

In this example, ink compositions were prepared having the components (pigment, purified water, water-miscible organic solvent, surfactant, and other additives) and contents shown in Tables 2 and 3 below. Specifically, the ink compositions of Examples 1 to 8 were prepared by mixing the components in Tables 2 and 3 below, stirring the mixture sufficiently in a stirrer for 30 minutes or more, and then filtering the mixture by using a 0.45 $\mu$m membrane filter. In this example, New Coccine (Aldrich), Brilliant Blue FCF (Aldrich), and Sunset yellow FCF (Aldrich) were used as a pigment component.

[Table 2]

| Component | | Example 1 | Example 2 | Example 3 | Example 4 |
|---|---|---|---|---|---|
| Pigment | SunSuet Yellow FCF | 4.5 g | - | - | 1.5 g |
| | New Coccine | - | 5g | - | 2 g |
| | Brilliant Blue FCF | - | - | 4 g | 2.5 g |
| Glycerin | | 12 g | 12 g | 12 g | 12 g |
| 1,2-Pentandiol | | 8 g | 8 g | 8 g | 8 g |

(continued)

| Component | Example 1 | Example 2 | Example 3 | Example 4 |
|---|---|---|---|---|
| **PEG-10 dimethicone (HLB 4.5)** | **0.2 g** | **0.2 g** | **0.2 g** | **0.2 g** |
| Phenoxyethanol | 0.1 g | 0.1 g | 0.1 g | 0.1 g |
| Purified water | 75.2 g | 74.7 g | 75.7 g | 73.7 g |

[Table 3]

| Component | | Example 5 | Example 6 | Example 7 | Example 8 |
|---|---|---|---|---|---|
| Pigment | SunSuet Yellow FCF | 4.5 g | - | - | 1.5 g |
| | New Coccine | - | 5g | - | 2 g |
| | Brilliant Blue FCF | - | - | 4 g | 2.5 g |
| Glycerin | | 12 g | 12 g | 12 g | 12 g |
| 1,2-Pentandiol | | 8 g | 8 g | 8 g | 8 g |
| **Glyceryl isostearate (HLB 3.8)** | | **0.2 g** | **0.2 g** | **0.2 g** | **0.2 g** |
| Phenoxyethanol | | 0.1 g | 0.1 g | 0.1 g | 0.1 g |
| Purified water | | 75.2 g | 74.7 g | 75.7 g | 73.7 g |

In addition, the ink compositions of Comparative Examples 1 to 8 having the components and contents shown in Tables 4 and 5 below were prepared in the same manner as above.

[Table 4]

| Component | | Comparative Example 1 | Comparative Example 2 | Comparative Example 3 | Comparative Example 4 |
|---|---|---|---|---|---|
| Pigment | SunSuet Yellow FCF | 4.5 g | - | - | **1.5** g |
| | New Coccine | - | 5g | - | 2g |
| | Brilliant Blue FCF | - | - | 4g | 2.5 g |
| Glycerin | | 12 g | 12 g | 12 g | 12 g |
| 1,2-Pentandiol | | 8 g | 8 g | 8 g | 8 g |
| **Surfynol 465 (HLB 13)** | | **0.2 g** | **0.2 g** | **0.2 g** | **0.2 g** |
| Phenoxyethanol | | 0.1 g | 0.1 g | 0.1 g | 0.1 g |
| Purified water | | 75.2 g | 74.7 g | 75.7 g | 73.7 g |

[Table 5]

| Component | | Comparative Example 5 | Comparative Example 6 | Comparative Example 7 | Comparative Example 8 |
|---|---|---|---|---|---|
| Pigment | SunSuet Yellow FCF | 4.5 g | - | - | 1.5 g |
| | New Coccine | - | 5g | - | 2g |
| | Brilliant Blue FCF | - | - | 4g | 2.5 g |
| Glycerin | | 12 g | 12 g | 12 g | 12 g |
| 1,2-Pentandiol | | 8 g | 8 g | 8 g | 8 g |
| **Polysorbate 20 (HLB 16.7)** | | **0.2 g** | **0.2 g** | **0.2 g** | **0.2 g** |
| Phenoxyethanol | | 0.1 g | 0.1 g | 0.1 g | 0.1 g |
| Purified water | 75.2 g | 74.7 g | 75.7 g | 73.7 g | |

**Experimental Example 1. Functional evaluation of ink compositions for printing surface of cosmetic**

In this experimental example, the ink compositions of Examples 1 to 8 and the ink compositions of Comparative Examples 1 to 8 were evaluated for functionality related to the efficacy of printing the surface of a cosmetic. Specifically, the evaluation of color development and tinting strength, the evaluation of caking phenomenon, the evaluation of stability in a sealed state, the evaluation of stability under room temperature dry conditions, and the evaluation of printing nozzle clogging phenomenon were sequentially performed.

**1-1. Evaluation of color development and tinting strength**

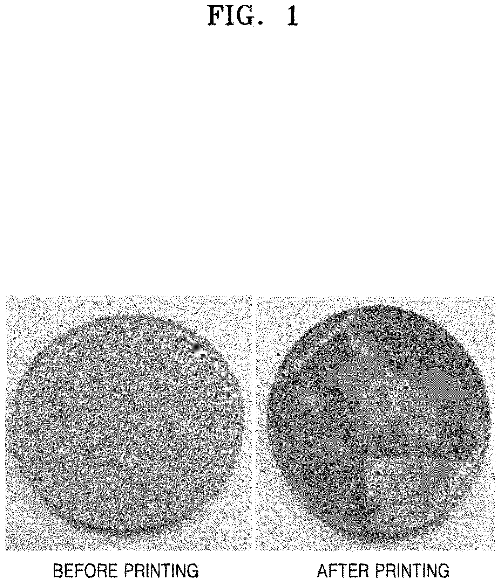

The surface of a cosmetic was printed in full color by using the ink compositions of Examples 1 to 8 through an inkjet printing method. Afterwards, the surface of the cosmetic printed with the ink composition as described above was visually confirmed.
As a result, as shown in FIG. 1, it was confirmed that when the surface of the cosmetic was printed by using the ink composition, vivid full-color printing was possible, and in particular, the surface of the cosmetic could be vividly printed with high color development or tinting strength.

**1-2. Evaluation of caking phenomenon**

The surface of a cosmetic was printed by using the ink compositions of Examples 1 to 8 and Comparative Examples 1 to 8, and was swept from side to side five times by using a brush, and then the presence and agglomeration (caking phenomenon) of the printed ink composition was evaluated according to the following criteria. The results are shown in Table 6 below.

@: After sweeping from side to side 5 times by using a brush, the patterns disappear and there are no agglomerates.
*100△: After sweeping from side to side 5 times by using a brush, the patterns and agglomerates remain.

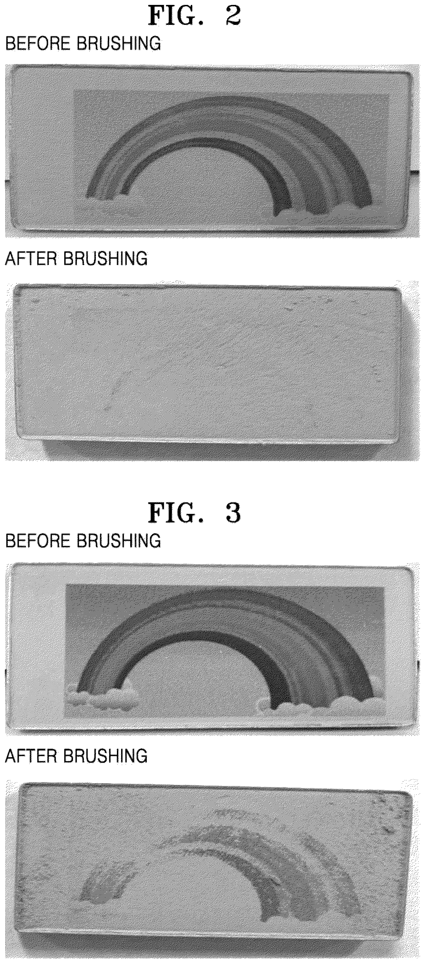

As a result, as shown in Table 6, Fig. 2 and Fig. 3, it was confirmed that on the surface of the cosmetic to which the ink compositions of the above examples were applied, after brushing, all patterns by the ink compositions disappeared and no agglomeration phenomenon occurred on the surface of the cosmetic. On the other hand, on the surface of the cosmetic to which the ink compositions of the above comparative examples including Surfynol 465 or Polysorbate 20 with a HLB value of 10 or more as a surfactant were applied, after brushing, patterns formed by the ink compositions were still observed and solid powder-like substances due to agglomeration were observed on part of the surface of the cosmetic.
Through this experimental example, it was confirmed that in the ink compositions of the above examples, by including a glyceryl ester-based surfactant (or a glyceryl fatty acid ester-based surfactant) or a silicone-based surfactant with a HLB value of 3 to 8 as a surfactant, the ink compositions could be printed very stably on the surface of a cosmetic (specifically, a cosmetic of a solid powder formulation or an oil-based cosmetic), as well as, in actually using the cosmetic after printing the ink compositions on the surface of the cosmetic, the ink compositions could be easily removed from the surface of the cosmetic, thereby causing no caking phenomenon such as agglomeration that occurs when components in the cosmetic and ink compositions become entangled. On the other hand, it was confirmed that when printing the surface of the cosmetic by using the ink compositions of the above comparative examples including a surfactant other than the glyceryl ester-based surfactant (or the glyceryl fatty acid ester-based surfactant) or the silicone-based surfactant with the HLB value of 3 to 8, in actually using the cosmetic, the ink compositions were not easily removed from the surface of the cosmetic and caused the caking phenomenon, resulting in deterioration in the usability and quality of the cosmetic. As a result, it was found that when printing the surface of a cosmetic by using the ink compositions of the above examples, the problems of feeling of use and quality deterioration due to the caking phenomenon caused in existing surface-printed cosmetics may be resolved.

## 1-3. Evaluation of stability in sealed state

100 ml of the ink compositions of Examples 1 to 8 and Comparative Examples 1 to 8 were each placed in heat-resistant glass bottles, and then each of glass bottles was sealed and stored in a 60°C constant temperature bath for 2 months. Afterwards, the presence or absence of sediment formed on the bottom of the bottles and layer separation were checked and evaluated according to the following criteria, and the results are shown in Table 6 below.

◎: No layer separation
○: Cloudy, but becomes transparent upon shaken
△: Cloudy and does not become transparent even when shaken
X: Complete layer separation

## 1-4. Evaluation of stability under room temperature dry conditions

The ink compositions of Examples 1 to 8 and Comparative Examples 1 to 8 were each refilled into ink cartridges and left in the air for 2 months without completely sealing the cartridge lid. An accelerated drying experiment at room temperature was performed and evaluated according to the following criteria, and the results are shown in Table 6 below.

@: No solid precipitation
O: Cloudy, but becomes transparent upon shaken
△: Cloudy and does not become transparent even when shaken
X: Complete layer separation

## 1-5. Evaluation of printing nozzle clogging phenomenon

The ink compositions of Examples 1 to 8 and Comparative Examples 1 to 8 were each refilled into ink cartridges and left at room temperature (25 °C) and low temperature (-18 °C) each for two weeks, the ink cartridges were mounted on a printer (Epson Stylus2880), and then printing was performed for nozzle evaluation. At this time, the number of nozzle cleaning times required to enable normal printing was measured and evaluated according to the following criteria, and the results are shown in Table 6 below.

◎: Normal printing is possible after one nozzle cleaning.
○: Normal printing is possible after nozzle cleaning 2 to 4 times.
△: Normal printing is possible after nozzle cleaning 5 to 10 times.
X: Normal printing is not possible even after nozzle cleaning 10 times.

## 1-6. Experiment result

Table 6 below shows the results of Experimental Examples 1-2 to 1-5 for each ink composition of Examples 1 to 8 and Comparative Examples 1 to 8.

[Table 6]

| | Caking phenomenon | Stability in sealed state | Stability under room temperature dry conditions | Printing nozzle clogging phenomenon |
|---|---|---|---|---|
| Example 1 | ◎ | ◎ | ◎ | ◎ |
| Example 2 | ◎ | ◎ | ◎ | ◎ |
| Example 3 | ◎ | ◎ | ◎ | ◎ |
| Example 4 | ◎ | ◎ | ◎ | ◎ |
| Example 5 | ◎ | ○ | ○ | ○ |
| Example 6 | ◎ | ○ | ○ | ○ |
| Example 7 | ◎ | ○ | ○ | ○ |
| Example 8 | ◎ | ○ | ○ | ○ |
| Comparative Example 1 | Δ | ○ | ◎ | ○ |
| Comparative Example 2 | Δ | ○ | ◎ | ○ |
| Comparative Example 3 | Δ | ○ | ◎ | ○ |
| Comparative Example 4 | Δ | ○ | ◎ | ○ |
| Comparative Example 5 | Δ | ○ | ◎ | ◎ |
| Comparative Example 6 | Δ | ○ | ◎ | ◎ |
| Comparative Example 7 | Δ | ○ | ◎ | ◎ |
| Comparative Example 8 | Δ | ○ | ◎ | ◎ |

As a result, as shown in Table 6, the ink composition according to one example hardly caused layer separation and precipitation of solids even after long-term storage, and did not cause clogging in the nozzle even when stored in the cartridge for a long period of time, which indicate that the stability under sealed/room temperature dry conditions and flowability within the nozzle were improved. In addition, it was confirmed that the caking phenomenon was also significantly improved when printing the ink composition according to one example on the surface of a cosmetic based on solid or wax components.

**Experimental Example 2. Confirmation of effect of ink composition on printing surface of cosmetic by treating surface of cosmetic with plasma**

**2-1. Confirmation of surface modification of cosmetic by plasma treatment**

In this experimental example, the oil-soluble surface of a cosmetic was subjected to atmospheric pressure plasma treatment to confirm whether the surface of the cosmetic was modified to be water-soluble.

The plasma generating power supply used was an AC high-voltage power supply (Uion, P15kVAC) with a frequency of 30 kHz and a maximum applied power of 2 kW. As a discharge condition, the applied power was 60 W, and the process was performed with a distance of 10 mm between the sample and the reactor. When argon is fixed at 3 L/min in the mixed gas flowing into the reactor in the process and the oxygen supply is 150 sccm, the mixing ratio is 5 %.

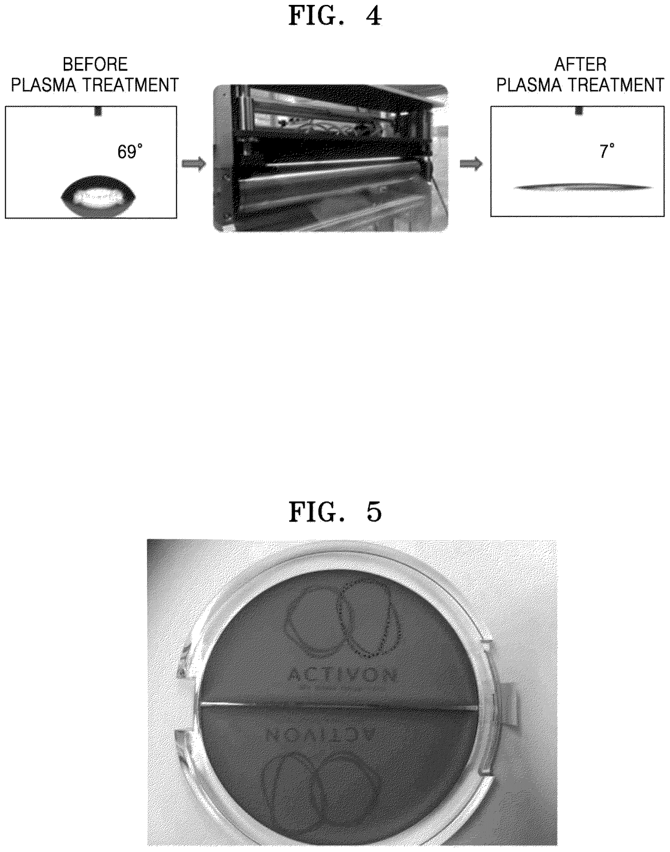

As a result, as shown in Fig. 4, it was confirmed that before atmospheric pressure plasma treatment, when the ink composition was dropped on the oil-soluble cosmetic surface, ink agglomeration occurred after printing due to a high contact angle, but after atmospheric pressure plasma treatment, when a water-soluble ink was dropped on the oil-soluble cosmetic surface, a low contact angle was formed due to the formation of radicals on the oil-soluble cosmetic surface and introduction of gases such as oxygen, nitrogen, and argon to the surface.

In other words, it was confirmed that by treating the surface of a cosmetic with plasma, the oil-soluble cosmetic surface may be modified to be hydrophilic, thereby enhancing the adhesion of water-soluble inks, preventing ink agglomeration phenomenon, and increasing color development or tinting strength.

## 2-2. Confirmation of effect of ink composition on printing surface of cosmetic by treating surface of cosmetic with plasma

In this experimental example, the effects of an ink composition on printing the surface of a cosmetic before and after subjecting the surface of the cosmetic to atmospheric pressure plasma treatment were compared.

As a result, as shown on the top of Fig. 5, it was confirmed that when printing the surface of the cosmetic was printed by using the ink composition before subjecting the surface to the atmospheric pressure plasma treatment, the ink composition did not adhere to the oil-soluble cosmetic surface and was separated therefrom, resulting in water droplet formation. On the other hand, as shown on the bottom of FIG. 5, it was confirmed that when printing the surface of the cosmetic was printed by using the ink composition after subjecting the surface to the atmospheric pressure plasma treatment, the ink composition completely adhered to the oil-soluble cosmetic surface, causing no water droplet formation and agglomeration at all.

Through the above experimental results, it was confirmed that the oil-soluble cosmetic surface may be modified to be water-soluble by subjecting the surface of the cosmetic to atmospheric pressure plasma treatment before printing the surface of the cosmetic. In addition, accordingly, it was confirmed that when the surface of the cosmetic was printed by using the ink composition, the separation phenomenon between the ink composition and the surface of the cosmetic, tinting or color development deterioration, agglomeration phenomenon, water droplet formation, and the like may be significantly reduced, thereby significantly improving the surface printability of the cosmetic.

Therefore, by plasma treating the surface of powder products such as powder pacts, eye shadows, or the like, and oil-soluble products such as lipsticks, sun sticks, or the like to make the surface hydrophilic, the effect of the ink composition on printing the surface of a cosmetic may be significantly improved.

## 2-3. Performance comparison between direct plasma and remote plasma

In this experimental example, the performance of direct plasma and remote plasma was compared in relation to surface modification of a cosmetic.

Specifically, before printing the surface of the cosmetic, the surface of the cosmetic was treated with direct plasma or remote plasma to compare the water-soluble modification level of the oil-soluble cosmetic surface, and after direct plasma or remote plasma treatment, the surface adhesion, tinting strength, color development, and the like of a water-soluble ink composition were compared when the surface of the cosmetic was printed with the water-soluble ink composition.

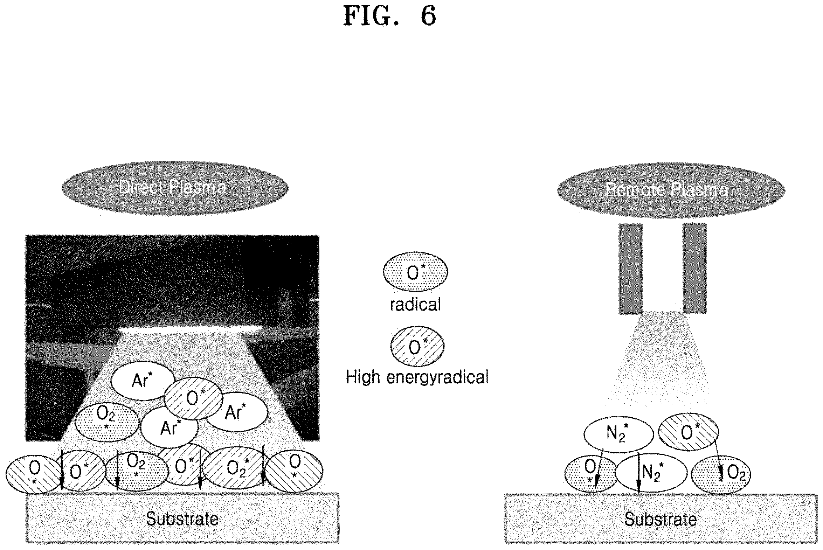

As a result, it was confirmed that compared to remote plasma treatment, when treated with direct plasma, the water-soluble modification of the oil-soluble cosmetic surface was significantly increased, and the surface adhesion, tinting strength, or color development of the water-soluble ink composition was significantly improved when the surface of the cosmetic was printed with the water-soluble ink composition. These results are understood to be a result of, as shown in Fig. 6, in direct plasma, the plasma being irradiated directly to the sample, so that the surface of the sample may retain a large amount of high-energy radicals, whereas in remote plasma, as plasma is not irradiated directly to the sample, the plasma is generated in the reactor and radicals are strongly blown onto the sample, and thus, in the case of remote plasma treatment, the high-energy radicals are lost before reacting with the sample, and accordingly, the effect of sample surface treatment is significantly reduced compared to direct plasma.

As the specific parts of the present disclosure have been described in detail above, it is clear to those skilled in the art that these specific descriptions are merely preferred embodiments and do not limit the scope of the present disclosure thereto. Accordingly, the substantial scope of the present disclosure will be defined by the appended claims and their equivalents.

## Claims

1. An ink composition for printing a surface of a cosmetic, the ink composition comprising:

   a water-soluble pigment; a water-miscible organic solvent; and a surfactant,
   wherein the surfactant is a glyceryl ester-based surfactant, a silicone-based surfactant, or a combination thereof.

2. The ink composition of claim 1, wherein the surfactant has a hydrophilic lipophilic balance (HLB) value of 3 to 8.

3. The ink composition of claim 1, wherein the water-miscible organic solvent is any one selected from the group consisting of an alkylene glycol having 2 to 5 carbon atoms, glycerin, a monoalcohol having 1 to 5 carbon atoms, an ether of an alkylene glycol having 2 to 5 carbon atoms, an ester of an alkylene glycol having 2 to 5 carbon atoms, a monooxyalkylenediol having 2 to 5 carbon atoms, a polyoxyalkylenediol having 5 to 10 carbon atoms, thiodiglycol, a monoalkanolamine, a dialkanolamine, a trialkanolamine, and combinations thereof.

4. The ink composition of claim 1, wherein the water-soluble pigment is any one selected from the group consisting of Sunset yellow FCF, New Coccine, Brilliant Blue FCF, and combinations thereof.

5. The ink composition of claim 1, wherein the surfactant is comprised in an amount of 0.05 to 0.5 wt%, based on a total weight of the ink composition.

6. The ink composition of claim 1, wherein the ink composition comprises 2 to 7 wt% of a water-soluble pigment, 10 to 25 wt% of a water-miscible organic solvent, and 0.05 to 0.5 wt% of a surfactant, based on a total weight of the ink composition.

7. The ink composition of claim 1, wherein the ink composition is applied to the surface of the cosmetic by using an inkjet printing method, and the ink composition applied to the surface of the cosmetic is removed from the surface of the cosmetic by 4 to 10 contacts.

8. The ink composition of claim 1, wherein the cosmetic is any one of a cosmetic of a solid powder formulation or an oil-based cosmetic.

9. A method of printing a surface of a cosmetic, comprising applying the ink composition of claim 1 to the surface of the cosmetic by using an inkjet printing method.

10. The method of claim 9, further comprising treating the surface of the cosmetic with plasma prior to the applying.

11. The method of claim 10, wherein the treating of the surface of the cosmetic with plasma is treating the surface of the cosmetic with atmospheric pressure plasma.

12. The method of claim 9, wherein the ink composition applied to the surface of the cosmetic is removed from the surface of the cosmetic by 4 to 10 contacts.

13. The method of claim 9, wherein the cosmetic is any one of a cosmetic of a solid powder formulation or an oil-based cosmetic.

FIG. 1

BEFORE PRINTING          AFTER PRINTING

# FIG. 2

BEFORE BRUSHING

AFTER BRUSHING

# FIG. 3

BEFORE BRUSHING

AFTER BRUSHING

# FIG. 4

BEFORE
PLASMA TREATMENT

69°

AFTER
PLASMA TREATMENT

7°

# FIG. 5

FIG. 6

## INTERNATIONAL SEARCH REPORT

| International application No. |
| --- |
| **PCT/KR2022/015375** |

| A. | CLASSIFICATION OF SUBJECT MATTER |
| --- | --- |

**C09D 11/32**(2014.01)i; **C09D 11/033**(2014.01)i; **C09D 11/10**(2006.01)i; **B41M 5/00**(2006.01)i; **A23P 20/00**(2016.01)i; **A61K 8/18**(2006.01)i

According to International Patent Classification (IPC) or to both national classification and IPC

| B. | FIELDS SEARCHED |
| --- | --- |

Minimum documentation searched (classification system followed by classification symbols)

C09D 11/32(2014.01); A61K 8/02(2006.01); A61K 8/19(2006.01); B41M 5/00(2006.01); C09D 11/00(2006.01); C09D 11/02(2006.01); C09D 11/30(2014.01); C09D 11/326(2014.01); C11D 17/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 화장품 (cosmetic), 인쇄 (printing), 잉크 (ink), 표면 (surface)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | KR 10-2004-0087095 A (AMOREPACIFIC CORPORATION) 13 October 2004 (2004-10-13)<br>See abstract; claims 1, 3, 5 and 10-13; and paragraphs [0025]-[0029] and [0038]-[0057]. | 1-3,7-9,12-13 |
| Y | | 4-6,10-11 |
| Y | JP 08-302255 A (SEIKO EPSON CORP.) 19 November 1996 (1996-11-19)<br>See abstract; claims 1 and 5; and paragraphs [0020]-[0026]. | 4-6 |
| Y | KR 10-2020-0077971 A (PLAMAX CO., LTD.) 01 July 2020 (2020-07-01)<br>See claim 1. | 10-11 |
| A | JP 2006-169301 A (UNION CHEMICAR CO., LTD. et al.) 29 June 2006 (2006-06-29)<br>See entire document. | 1-13 |
| A | KR 10-2019-0129977 A (SHISEIDO COMPANY, LIMITED) 20 November 2019 (2019-11-20)<br>See entire document. | 1-13 |

| ☐ Further documents are listed in the continuation of Box C. | ☑ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **19 January 2023** | **20 January 2023** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

| International application No. |
| --- |
| **PCT/KR2022/015375** |

| Patent document cited in search report | | | Publication date (day/month/year) | Patent family member(s) | | | Publication date (day/month/year) |
| --- | --- | --- | --- | --- | --- | --- | --- |
| KR | 10-2004-0087095 | A | 13 October 2004 | KR | 10-0500588 | B1 | 18 July 2005 |
| JP | 08-302255 | A | 19 November 1996 | None | | | |
| KR | 10-2020-0077971 | A | 01 July 2020 | None | | | |
| JP | 2006-169301 | A | 29 June 2006 | None | | | |
| KR | 10-2019-0129977 | A | 20 November 2019 | CN | 110461958 | A | 15 November 2019 |
| | | | | EP | 3604460 | A1 | 05 February 2020 |
| | | | | JP | 06-386613 | B1 | 05 September 2018 |
| | | | | JP | 2018-168233 | A | 01 November 2018 |
| | | | | US | 2020-0095441 | A1 | 26 March 2020 |
| | | | | WO | 2018-180732 | A1 | 04 October 2018 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020210164872

- KR 102035916

**Non-patent literature cited in the description**

- **W. C. GRIFFIN.** *J. Soc. Cosmetic Chem.,* 1954, vol. 5, 249

- **K. H. SCHRADER.** Grundlagen und Rezepturen der Kosmetika [Fundamentals and formulations of cosmetics. Huethig Verlag, 1989